# EUROPÄISCHE PATENTSCHRIFT

(11) **EP 3 937 832 B1**
(45) Veröffentlichungstag und Bekanntmachung des Hinweises auf die Patenterteilung: **01.05.2024**
(21) Anmeldenummer: 20709603.3
(22) Anmeldetag: 13.03.2020
(51) Int. Cl.: A61C 8/00, A61B 17/88, A61B 90/00, A61C 1/18

(54) **DENTALMEDIZINISCHER DREHMOMENTSCHLÜSSEL**
DENTAL TORQUE WRENCH
CLÉ DYNAMOMÉTRIQUE MÉDICO-DENTAIRE

(30) Priorität: 13.03.2019 EP 19162550
(43) Veröffentlichungstag der Anmeldung: 19.01.2022
(60) Teilanmeldung: 24158725.2
(73) Patentinhaber: Straumann Holding AG, 4002 Basel (CH)
(72) Erfinder: MAURY, Damien, 2613 Villeret (CH); COURVOISIER, Stéphane, 2613 Villeret (CH)
(74) Vertreter: Schaad, Balass, Menzl & Partner AG
(86) Internationale Anmeldenummer: PCT/EP2020/056753
(87) Internationale Veröffentlichungsnummer: WO 2020/182973

(56) Entgegenhaltungen:
- DE-A1-102006 040 809
- DE-A1-102017 107 278
- TW-B- I 595 864

## Beschreibung

Die Erfindung betrifft einen Drehmomentschlüssel für die Medizinaltechnik, insbesondere die Dentalmedizin.

Aus der Dentalmedizin ist bekannt, Implantate in den Kieferknochen einzusetzen und an den Implantaten Verbindungselemente, wie Abutments, zu befestigen, auf welche dann die Suprastruktur, insbesondere eine Krone oder eine Brücke, aufgesetzt wird. Zum Einschrauben des Implantats bzw. des Verbindungselements kann ein Eindrehinstrument formschlüssig aufgesetzt und über einen Drehmomentschlüssel gedreht werden. Je nach Zahnimplantatsystem sind Drehmomente im Bereich von 10 Ncm bis 100 Ncm typischerweise erforderlich.

Zur Anbringung eines Drehmoments über einen breiten Bereich, beispielsweise von 10 Ncm bis 100 Ncm, können mehrere, jeweils für einen Teilbereich des breiten Bereichs ausgelegte Drehmomentschlüssel nacheinander verwendet werden. Die Verwendung mehrerer Drehmomentschlüssel ist jedoch nachteilig, weil die Kosten entsprechend der Anzahl der Drehmomentschlüssel multipliziert werden. Ferner ist die Handhabung mehrerer Drehmomentschlüssel während einer Behandlung unpraktisch.

Alternativ kann ein Drehmomentschlüssel vorteilhaft verwendet werden, welcher zur Anbringung eines Drehmoments über den ganzen breiten Bereich geeignet ist.

Aus DE 10 2006 040809 A1 ist ein Drehmomentinstrument mit einer Ratschenfunktion zur Anwendung in der Dentalmedizin bekannt, welches einen Griff und einen Kopf umfasst, wobei der Kopf eine Ausnehmung zur Aufnahme eines Eindrehmittels aufweist. Innerhalb der Ausnehmung befindet sich eine Sperrklinke, welche mit dem Eindrehmittels in Eingriff steht, um in eine vorgegebene Drehrichtung ein Drehmoment auf das Implantat um dessen Längsachse auszuüben. Im Griff ist ein zentraler Biegestab angeordnet, welcher an dem dem Kopf gegenüberliegenden Ende des Griffes aus dem Griff herausragt. Der Biegestab ist in einer axialen Bohrung innerhalb des Griffs an seinem innenliegenden Ende axial festgelegt. Der Biegestab ist mittels einer Gewindeverbindung drehfest mit einem Bolzen verbunden, welcher an seinem bis zur Ausnehmung reichenden Ende die Sperrklinke aufweist. Der Bolzen ist mittels einer Lagervorrichtung im Griff und Kopf drehbar gelagert, sodass die Ausrichtung der Sperrklinke durch Drehen des Biegestabs in die gewünschte Richtung zur Einleitung eines Drehmoments gedreht werden kann. Weiter enthält der Griff eine seitliche, nach aussen offene Nut, welche nach innen bis zur Bohrung reicht. Die Nut enthält achsparallele Seitenwände und erstreckt sich vom freien Ende über im Wesentlichen die halbe Gesamtlänge des Griffs. Die Nut bildet somit eine in den Griff integrierte Führung für den zentral und fest im Griff montierten Biegestab. Am Ende besitzt der Griff radiale Ansätze, zwischen welchen sich gleichfalls die Nut erstreckt. Wenigstens einer der radialen Ansätze enthält eine Drehmomentskala, gemäss welcher die Auslenkung des Biegestabs und somit das eingeleitete Drehmoment erfassbar ist. In einer Ausführungsform ist ein Element zur Erfassung und/oder Anzeige des maximal eingeleiteten Drehmoments einem der radialen Ansätze angeordnet. Das Element ist hier als ein mittels des Biegestabs entsprechend dem ausgeübten Drehmoment radial verschiebbarer Ring ausgebildet.

Aus TW I 595 864 B ist ein Drehmomentinstrument mit einer Ratschenfunktion zur Anwendung in der Dentalmedizin bekannt, welches einen Kopfbereich, einen am Kopfbereich befestigten Schaftbereich und einen an den Kopfbereich anschliessenden Betätigungshebel umfasst. Der Betätigungshebel weist einen sich in Längsrichtung des Drehmomentinstruments erstreckenden Schlitz auf, in welchem der Schaftbereich in Ruheposition gelagert ist und welcher eine Bewegung des Betätigungshebels in Umfangsrichtung bezüglich einer in den Kopfbereich ausgebildeten Dreheinheit ermöglicht. Der Schaftbereich umfasst einen Indikatorbereich und eine sich in Umfangsrichtung bezüglich der Dreheinheit in den Indikatorbereich erstreckende Aussparung. Die Aussparung ist dazu bestimmt, ein Anschlagselement aufzunehmen und dessen lösbare Befestigung in einer vorbestimmten Position entlang der Aussparung zu ermöglichen. Vor der Bedienung des Drehmomentinstruments wird das Anschlagselement in der Aussparung in einer Position lösbar befestigt, welche einem auszuübenden Drehmoment entspricht. Anschliessend wird eine Kraft auf den Betätigungshebel in Anzugsrichtung ausgeübt, bis er in Anlage mit dem Anschlagselement kommt und das auszuübende Drehmoment erreicht ist.

Ein Drehmomentschlüssel mit einer Ratschenfunktion zur Anwendung in der Dentalmedizin ist in der EP 0 704 281 A1 offenbart. Der Drehmomentschlüssel umfasst ein Drehmomentinstrument, insbesondere ein Ratscheninstrument, sowie einen daran als Zubehör ansetzbaren Drehmomentindikator. Der Drehmomentindikator besitzt einen hülsenförmigen, auf einen Griff des Drehmomentinstruments aufschiebbaren Träger, an dem ein elastischer Biegestab befestigt ist. Auf das freie Ende des Biegestabes wird bei Betätigung eine Kraft in Anzugsrichtung des Drehmomentschlüssels ausgeübt und das erzeugte Drehmoment an einer Masseinteilung angezeigt. Die Reinigung und die Sterilisation dieses Drehmomentschlüssels erfordern eine Demontage des Drehmomentschlüssels und sind somit relativ aufwendig. Ferner ist dieser Drehmomentschlüssel für einen fixen Drehmomentbereich von 0 bis 40Ncm ausgelegt. Ein höherer Drehmomentbereich kann somit mit einem weiteren, für einen höheren Drehmomentbereich fix voreingestellten Drehmomentschlüssel gemessen werden. Dies ist jedoch nachteilig, wie dies bereits erwähnt wurde. Alternativ kann der fix voreingestellte Drehmomentbereich von 0 bis 40Ncm auf einen breiteren Bereich angepasst werden, beispielsweise durch die Zulassung einer grösseren Auslenkung des Biegestabs. Diese Lösung hat allerdings ein Problem, weil der Raum im Mundbereich begrenzt ist und eine ausreichende Auslenkung nur schwer erreicht werden kann. Alternativ kann einen biegesteiferen Biegestab verwendet werden, welcher jedoch mit nachteiligen Auswirkungen auf die Ablesegenauigkeit verbunden ist.

Aus der DE 20 2004 014 195 U1 ist eine Ausführungsform eines Drehmomentschlüssels für die Medizinaltechnik bekannt, welche einen zuvorderst liegenden Kopfbereich, einen anschliessenden Halsbereich, dem ein Schaftbereich folgt, einen an den Schaftbereich anschliessenden Indikatorbereich und einen zuhinterst angeordneten Griffbereich umfasst. Der Drehmomentschlüssel umfasst ferner eine im Kopfbereich vorgesehene Aufnahmeöffnung zur Aufnahme eines Eindrehinstruments. Weiter umfasst der Drehmomentschlüssel einen sich vom Halsbereich erstreckenden Betätigungshebel, welcher zuerst einen vom Halsbereich längs des Schaftbereichs verlaufenden Biegeabschnitt aufweist. Der Betätigungshebel endet frei mit einem dem Biegeabschnitt folgenden biegefesten Griffteil, welcher entlang des Griffbereichs verläuft. Der Indikatorbereich ist durch einen U-förmigen Bügel gebildet, dessen Öffnung dem Betätigungshebel zugewandt ist und welcher einen Freiraum umschliesst. Der Griffteil weist einen Ast auf, welche in den Freiraum hineinragt. Ferner umfasst der Bügel eine Schulter, mit der ein am Ende des Asts ausgebildetes Anschlagstück in Anlage kommt, wenn die Auslenkung des Betätigungshebels ein vorbestimmtes Mass erreicht. Dieser Drehmomentschlüssel ist für einen fixen Drehmomentbereich von 0 bis 40Ncm ausgelegt. Ähnlich wie bei der vorstehend bereits beschriebenen Ausführungsform der EP 0 704 281 A1 könnte der fix vorbestimmte Drehmomentbereich auf einen breiteren Bereich angepasst werden, sodass eine grössere Auslenkung des Betätigungshebels möglich ist. Diese Lösung ist jedoch ebenfalls mit dem gleichen Problem des begrenzten Raums im Mundbereich konfrontiert. Alternativ könnte einen biegesteiferen Betätigungshebel verwendet werden, welcher jedoch mit nachteiligen Auswirkungen auf die Ablesegenauigkeit verbunden wäre.

Aufgabe der vorliegenden Erfindung ist es somit, einen Drehmomentschlüssel für die Medizinaltechnik, insbesondere für die Dentalmedizin, zur Verfügung zu stellen, welcher eine sichere Bedienung ermöglicht und mit welchem ein Drehmoment in einem breiten Drehmomentbereich bei einem reduzierten Platzbedarf aufgebracht werden kann.

Diese Aufgabe wird erfindungsgemäss mit einem Drehmomentschlüssel gemäss Anspruch 1 gelöst. Bevorzugte Ausführungsformen der Erfindung sind in den abhängigen Ansprüchen wiedergegeben.

Die Erfindung betrifft einen Drehmomentschlüssel für die Medizinaltechnik, insbesondere die Dentalmedizin, mit einem eine Aufnahmeöffnung aufweisenden Kopfbereich, welche Aufnahmeöffnung eine Rotationsachse definiert und dazu bestimmt ist, ein Eindrehinstrument in der Erstreckung der Rotationsachse aufzunehmen.

Ferner weist der Drehmomentschlüssel einen an den Kopfbereich anschliessenden, sich wenigstens annähernd in einer rechtwinklig zur Rotationsachse verlaufenden Ebene erstreckenden, eine Längsachse des Drehmomentschlüssels definierenden, wenigstens annähernd biegefesten Schaftbereich auf.

Weiter umfasst der Drehmomentschlüssel einen Betätigungshebel und ein am Schaftbereich ausgebildetes Auflager.

In einer bevorzugten Ausführungsform sind der Kopfbereich, der Schaftbereich und das Auflager integral einstückig ausgebildet, um Schnittstellen zu vermeiden, welche die Reinigung des Drehmomentschlüssels erschweren.

Der Betätigungshebel weist einen elastisch biegbaren Biegeabschnitt und einen an den Biegeabschnitt anschliessenden Griffabschnitt auf. Mit dem elastisch biegbaren Biegeabschnitt wird sichergestellt, dass eine Biegung reversibel bleibt und der Betätigungshebel seine ursprüngliche Form wieder aufnimmt, wenn keine Kraft mehr aufgebracht wird. Somit kann die Anzeigegenauigkeit des aufgebrachten Drehmoments über die Lebensdauer des Drehmomentschlüssels gewährleistet sein.

In einer bevorzugten Ausführungsform ist der Biegeabschnitt linearelastisch biegbar. Ein linearelastisch biegbarer Biegeabschnitt weist eine proportional zur einwirkenden Kraft resultierende Biegung auf, welche eine praktische Bedienung des Drehmomentschlüssels ermöglicht.

Vorzugsweise bildet der Griffabschnitt das freie Ende des Betätigungshebels. In einer bevorzugten Ausführungsform schliesst der Griffabschnitt direkt an den Biegeabschnitt an. Der Griffabschnitt kann eine Verdickung aufweisen, um einen besseren Griff des Betätigungshebels zu ermöglichen und den Griffabschnitt biegesteif zu machen.

Der Betätigungshebel ist mit einem dem Griffabschnitt abgewandten Endbereich zwischen dem Auflager und dem Kopfbereich über einen Befestigungspunkt des Betätigungshebels am Schaftbereich befestigt. Konkret kann aber der Befestigungspunkt einen Abschnitt des Endbereichs darstellen, bspw. wenn der Endbereich in einem Spalt des Schaftbereichs eingeklemmt ist. Vorzugsweise ist der Betätigungshebel mit dem diesseitigen Endbereich des Biegeabschnitts am Schaftbereich befestigt, um eine einfache Konstruktion des Betätigungshebels zu erlauben.

Es ist jedoch auch möglich, den diesseitigen Endbereich des Betätigungshebels steif vorzusehen. Zwischen dem steifen Endbereich und dem Griffabschnitt liegt der Biegeabschnitt. Das Auflager ist derart positioniert, dass bei der Ausübung einer in Anzugsrichtung gerichteten Kraft auf den Betätigungshebel der Biegeabschnitt in Anlage mit dem Auflager kommt. Diese Anordnung ermöglicht eine Biegung des Betätigungshebels erst nach dem steifen Endbereich, d.h. ab einem weiter in Richtung zum Griffabschnitt angeordneten Abschnitt des Betätigungshebels. Mit unterschiedlichen Längen des steifen Endbereichs können die Biegeeigenschaften des Betätigungshebels angepasst werden, sodass die Auslenkung des Betätigungshebels entsprechend gestaltet werden kann. Diese Möglichkeit kann vorteilhaft sein, wenn der um den Schaftbereich herum für eine Auslenkung des Betätigungshebels verfügbare Platz auf den dem Kopfbereich zugewandten Endbereich des Schaftbereichs begrenzt ist.

In seiner Ruheposition, d.h. wenn keine Kraft auf den Betätigungshebel ausgeübt wird, ist der Betätigungshebel, in Umfangsrichtung bezüglich der Rotationsachse gesehen, vom Auflager beabstandet.

Vorzugsweise liegt der Betätigungshebel in seiner Ruheposition parallel zur Längsachse, d.h. der Betätigungshebel ist parallel zum Schaftbereich angeordnet, um eine kompakte Ausführungsform zu ermöglichen.

In einer bevorzugten Ausführungsform ist der Betätigungshebel in seiner Ruheposition radial zur Rotationsachse angeordnet. Auf diese Weise stimmt der Angriffspunkt des Drehmoments wenigstens annähernd mit dem Mittelpunkt der Aufnahmeöffnung überein. Folglich kann ein unerwünschter weiterer Hebelarm zwischen dem Angriffspunkt des Drehmoments und dem Mittelpunkt der Aufnahmeöffnung minimiert werden. Somit kann sichergestellt werden, dass beim Drehen das Eindrehinstrument wenigstens annähernd parallel zur Rotationsachse bleibt.

Alternativ kann der Betätigungshebel in seiner Ruheposition, in Längsrichtung gesehen, weg vom Kopfbereich radial versetzt zur Rotationsachse und schräg zur Längsrichtung angeordnet sein.

In einer bevorzugten Ausführungsform ist der Befestigungspunkt des Betätigungshebels, in Längsrichtung gesehen und gemessen ab dem dem Kopfbereich zugewandten Ende des Drehmomentschlüssels, zwischen einem Drittel und der Hälfte der Länge des Drehmomentschlüssels angeordnet. Diese Anordnung ermöglicht eine Auslenkung des Betätigungshebels ausserhalb des Mundes des Patienten, sodass der Drehmomentschlüssel bequem bedient werden kann.

Bei der Aufbringung einer Kraft in einer Anzugsrichtung auf den Betätigungshebel, vorzugsweise auf den Griffabschnitt, gelangt der Betätigungshebel in eine Auslenkposition. Bei in die Aufnahmeöffnung eingesetztem Eindrehinstrument überträgt der Betätigungshebel durch Mitnahme des Eindrehinstruments durch den Kopfbereich somit ein Drehmoment ans Eindrehinstrument.

Der Abstand, in Umfangsrichtung bezüglich der Rotationsachse gesehen, zwischen dem sich in Ruheposition befindenden Betätigungshebel und dem Auflager ist derart bemessen, dass beim Erreichen einer Referenzkraft der ausgelenkte Betätigungshebel am Auflager in Anlage kommt.

Der Referenzkraft entspricht ein Referenzdrehmoment, welches auf das Eindrehinstrument ausgeübt wird.

Erfindungsgemäss kommt der Biegeabschnitt beim Erreichen der Referenzkraft am Auflager in Anlage. Ferner ist der Biegeabschnitt bei der Aufbringung einer in Anzugsrichtung gerichteten, die Referenzkraft überschreitenden Kraft weiter in Anzugsrichtung biegbar.

Vorzugsweise ist das Auflager fest am Schaftbereich ausgebildet, d.h. das Auflager ist nicht beweglich. Somit kann das Auflager als ein fester Stützpunkt zum Biegen des Betätigungshebels verwendet werden, wenn der Betätigungshebel in Anlage mit dem Auflager kommt und eine Kraft auf den Betätigungshebel weiter in Anzugsrichtung ausgeübt wird.

In einer bevorzugten Ausführungsform ist das Auflager einstückig und integral mit dem Schaftbereich ausgebildet, um Schnittstellen zu vermeiden und die Reinigung des Drehmomentschlüssels zu vereinfachen.

Es ist auch möglich, das Auflager lösbar befestigbar am Schaftbereich auszubilden. Beispielsweise kann das Auflager in der Form eines Rings oder eines Gleitteils ausgebildet sein, der bzw. das entlang dem Schaftbereich in Längsrichtung verschiebbar ist und durch ein Befestigungsmittel wie eine Schraube oder einen Stift an dem Schaftbereich lösbar befestigt werden kann. Folglich kann die Position des Auflagers entlang dem Schaftbereich gewählt werden und somit kann der Drehmomentbereich des Drehmomentschlüssels flexibel eingestellt werden.

Bei der Ausübung einer in Anzugsrichtung gerichteten Kraft auf den Betätigungshebel, vorzugsweise auf den Griffabschnitt, verformt sich der Biegeabschnitt des Betätigungshebels bogenförmig und wird der Betätigungshebel von seiner Ruheposition relativ zum Schaftbereich in Anzugsrichtung ausgelenkt.

Der Biegeabschnitt kann in einen ersten Bogenabschnitt und einen zweiten Bogenabschnitt unterteilt werden. Der erste Bogenabschnitt verläuft von dem dem Kopfbereich zugewandten Ende des Biegeabschnitts bis zu einem Kontaktpunkt des Biegeabschnitts mit dem Auflager. Der zweite Bogenabschnitt verläuft von dem Kontaktpunkt bis zu dem dem Kopfbereich abgewandten Ende des Biegeabschnitts.

Wenn eine in Anzugsrichtung gerichtete, die Referenzkraft überschreitende Kraft ausgeübt wird, ändert sich das Biegeverhalten des Biegeabschnitts. Der erste Bogenabschnitt behält annähernd die gleiche Biegung wie beim Erreichen des Auflagers, unabhängig von der die Referenzkraft überschreitende ausgeübten Kraft. Hingegen kann sich der zweite Bogenabschnitt weiter in Anzugsrichtung in Abhängigkeit von der die Referenzkraft überschreitende ausgeübten Kraft verformen und biegen.

Somit ist der für die Auslenkung des ersten Bogenabschnitts benötigte Raum bei der Ausübung der die Referenzkraft überschreitenden Kraft kleiner als bei gattungsgemässen Drehmomentschlüsseln. Dies ist vorteilhaft, wenn der Raum im Mundbereich begrenzt ist und folglich nur wenig Platz für die Auslenkung des ersten Bogenabschnitts vorhanden ist. Ferner kann das der Referenzkraft überschreitenden Kraft entsprechende Drehmoment durch einfaches Ablesen auf dem Indikatorbereich weiter in einer kontrollierbaren Weise ausgeübt werden, im Gegensatz zu den gattungsgemässen Drehmomentschlüsseln. Der zweite Bogenabschnitt kann nämlich weiter gebogen werden, sodass die Auslenkung des zweiten Bogenabschnitts im Indikatorbereich abgelesen werden kann.

In einer bevorzugten Ausführungsform ist der Betätigungshebel derart ausgebildet, dass er beim Erreichen der Referenzkraft wenigstens annähernd parallel zur Längsrichtung angeordnet ist und eine Referenzposition annimmt. Ferner kann die Auslenkung des Betätigungshebels von der Ruheposition bis zur Referenzposition und von der Referenzposition bis zu einer maximal ausgelenkten Position, welche einer maximal auszuübenden Kraft entspricht, in etwa gleich langen Abschnitten verlaufen, welche sich in Umfangsrichtung bezüglich der Rotationsachse erstrecken. Somit kann der Drehmomentschlüssel kompakt entlang der Längsachse ausgebildet sein. Ferner kann der Drehmomentschlüssel durch eine etwa gleich lange Auslenkung des Betätigungshebels im Kraftbereich unter und über der Referenzkraft einfach bedient werden.

Die Position des Auflagers, in Längsrichtung gesehen, auf dem Schaftbereich kann als weiterer Parameter verwendet werden, um die Konstruktion des Drehmomentschlüssels zu bestimmen.

In einer bevorzugten Ausführungsform ist das Auflager, in Längsrichtung gesehen, zwischen ein Viertel und zwei Drittel der Länge des Betätigungshebels, in Längsrichtung gesehen und gemessen ab dem dem Kopfbereich zugewandten Ende des Betätigungshebels angeordnet. Diese Anordnung des Auflagers ermöglicht eine Auslenkung des Betätigungshebels im Indikatorbereich, welche eine feine Einstellung des ausgeübten Drehmoments erlaubt. Bevorzugt ist das Auflager etwa mittig zum Biegeabschnitt angeordnet, um eine gute Kraftverteilung zu ermöglichen. Besonders bevorzugt ist das Auflager etwa um ein Drittel der Länge des Biegeabschnitts, in Längsrichtung gesehen und gemessen ab dem dem Kopfbereich zugewandten Ende des Biegeabschnitts, angeordnet. Diese Anordnung ermöglicht erfahrungsgemäss eine Optimierung der Einstellung des ausgeübten Drehmoments.

In einer bevorzugten Ausführungsform ist der Biegeabschnitt durch einen Biegestab gebildet. Vorzugsweise ist der Querschnitt des Biegestabs gleich über die ganze Länge des Biegestabs. Besonders bevorzugt ist der Querschnitt kreisförmig. Diese Anordnung ermöglicht eine einfache Konstruktion.

Erfindungsgemäss weist der Schaftbereich eine parallel zur Ebene verlaufende Aussparung auf, in welcher der Betätigungshebel verläuft und welche einen Freiraum für die Auslenkung des Betätigungshebels bildet. Somit kann eine kompakte Ausführungsform des Drehmomentschlüssels erzielt werden. Vorzugsweise gehört der Boden der Aussparung zur Oberfläche des Drehmomentschlüssels, um eine einfache Reinigung zu ermöglichen.

Vorzugsweise ist ein Spalt zwischen dem Betätigungshebel und dem Boden der Aussparung vorgesehen, um eine einfache Reinigung zu ermöglichen.

Erfindungsgemäss weist der Schaftbereich eine sich in Längsrichtung erstreckende, in Anzugsrichtung gesehen, dem Betätigungshebel vorlaufende Wand auf, welche die Aussparung begrenzt. Die Wand kann sich somit im Wesentlichen in Richtung zum Indikatorbereich erstrecken. Ferner ist das Auflager durch eine konkav zur Rotationsachse geformte, in die Ebene verlaufende Krümmung der Wand ausgebildet. Beim Erreichen der Referenzkraft kommt der ausgelenkte Betätigungshebel an dem Kontaktpunkt in Anlage mit der Krümmung. Folglich ist das Auflager im Schaftbereich integriert und bildet das Auflager keine Unebenheit auf der Oberfläche des Drehmomentschlüssels, welche im Mundbereich einhaken könnte. Somit ist die Bedienung des Drehmomentschlüssels sicher.

In einer bevorzugten Ausführungsform bildet ein dem freien Ende des Betätigungshebels zugewandter Endbereich der Wand das Auflager. Alternativ kann das Auflager durch eine an der Wand ausgebildete, sich parallel zur Ebene auf der der Aussparung zugewandten Seite der Wand erstreckende Verdickung ausgebildet sein. Vorzugsweise ist die Verdickung am Endbereich der Wand ausgebildet.

Der Querschnitt des Schaftbereichs in seinem zwischen dem Befestigungspunkt des Betätigungshebels und dem Auflager verlaufenden Abschnitt kann somit etwa U-förmig ausgebildet sein, wobei der Boden der U-Form den Boden der Aussparung bildet. Von seiner Ruheposition bis zu einer ausgelenkten Position bewegt sich der Betätigungshebel, in Querschnitt gesehen, in den Freiraum zwischen den zwei Schenkeln der U-Form. Weiter kann der Querschnitt des Schaftbereichs in seinem zwischen dem Auflager und dem dem Indikatorbereich zugewandten Ende des Schaftbereichs verlaufenden Abschnitt L-förmig ausgebildet sein, wobei ein Schenkel der L-Form den Boden der Aussparung bildet und der weitere Schenkel der L-Form dem Betätigungshebel, in Anzugsrichtung gesehen, nachlaufend ausgebildet ist.

In einer bevorzugten Ausführungsform kann der Schaftbereich einen sich in Längsrichtung erstreckenden, an den Kopfbereich anschliessenden ersten Abschnitt und einen an den ersten Abschnitt anschliessenden, sich in der Ebene aufweitend ausgebildeten zweiten Abschnitt umfassen. Der Querschnitt des ersten Abschnitts ist, in der Ebene gemessen, schmaler als der Querschnitt des zweiten Abschnitts gestaltet. Folglich kann der schmale erste Bereich bequem in den Mundraum bewegt werden, wobei der breite zweite Abschnitt ausserhalb des Mundraums liegt und eine sichere Handhabung ermöglicht. Vorzugsweise stellt der erste Abschnitt ein Drittel und der zweite Abschnitt zwei Drittel des Schaftbereichs dar, um eine optimale Handhabung sicherzustellen.

In einer bevorzugten Ausführungsform kann sich der zweite Abschnitt schräg zur Längsrichtung erstrecken.

In einer bevorzugten Ausführungsform kann für den Betätigungshebel ein anderes Material, beispielsweise Federstahl, als für den Schaftbereich verwendet werden, sodass die Biegeeigenschaften des Betätigungshebels unabhängig von den Eigenschaften des Schaftbereichs gestaltet werden können. In diesem Fall wird der Betätigungshebel separat hergestellt und am Schaftbereich während der Montage des Drehmomentschlüssels befestigt. Die Befestigung des Betätigungshebels kann beispielsweise durch Klemmen, Schweissen oder ein Schraubsystem an einem Befestigungspunkt des Schaftbereichs erfolgen. Es ist jedoch auch möglich, den Betätigungshebel einstückig und integral mit dem Schaftbereich auszubilden, um Schnittstellen zu vermeiden und die Reinigung des Drehmomentschlüssels zu vereinfachen.

In einer bevorzugten Ausführungsform weist der Kopfbereich eine Ratschenfunktion auf. Das heisst, der Kopfbereich ermöglicht einen Freilauf des eingesetzten Eindrehinstruments bei einer Drehung entgegen der Anzugsrichtung. Ratschenfunktionen sind dem Fachmann bekannt. Sie erlauben das Ein- und/oder Ausschrauben ohne den Drehmomentschlüssel wiederholt vom Eindrehinstrument zu lösen und wiederholt auf das Eindrehinstrument aufzusetzen. Somit wird eine praktische Bedienung des Drehmomentschlüssels sichergestellt.

In einer bevorzugten Ausführungsform umfasst der Drehmomentschlüssel einen vom freien Endbereich des Schaftbereichs abstehenden Indikatorbereich, wobei der Betätigungshebel sich mindestens bis zum Indikatorbereich erstreckt. Der Indikatorbereich liegt somit an dem dem Kopfbereich abgewandten Ende des Schaftbereichs und erstreckt sich vorzugsweise wenigstens annähernd parallel zur Ebene, beispielsweise in der Form eines Plättchens.

Besonders bevorzugt ist der Indikatorbereich einstückig und integral mit dem Schaftbereich ausgebildet. Diese Ausführungsform ist konstruktiv besonders einfach und ihre Herstellung kostengünstig.

Bevorzugt erstreckt sich der Betätigungshebel bis in den Indikatorbereich hinein, um eine Überlappung des Betätigungshebels und des Indikatorbereichs zu haben. Somit kann die relative Position des Betätigungshebels bezüglich des Indikatorbereichs besser bestimmt werden.

Besonders bevorzugt erstreckt sich der Betätigungshebel über den Indikatorbereich hinaus, um eine einfache Bedienung des Betätigungshebels über dessen dem Indikatorbereich hinausgehenden Abschnitt zu ermöglichen.

Bei der Aufbringung der Kraft in Anzugsrichtung auf den Betätigungshebel ist die Auslenkung des Betätigungshebels, ausgehend von dessen Ruheposition, ein Mass für das generierte Drehmoment.

In einer bevorzugten Ausführungsform sind auf dem Indikatorbereich Messmarkierungen angebracht, an welchen die Auslenkung des Betätigungshebels als erzeugtes Drehmoment ablesbar ist.

In einer bevorzugten Ausführungsform wird der Betätigungshebel bei seiner Auslenkung durch ein Führungselement in einer parallel zur Ebene verlaufenden Auslenkungsebene bewegbar gehalten. Somit wird sichergestellt, dass der Betätigungshebel in der Auslenkungsebene eine relative Position bezüglich der Rotationsachse behält, welche die Ausübung einer Kraft schräg zur Auslenkungsebene verhindert. Folglich bleibt die Drehmomentachse wenigstens annähernd parallel zur Rotationsachse.

In einer bevorzugten Ausführungsform kann der Indikatorbereich zusammen, d.h. integral mit dem Führungselement ausgebildet sein. Somit ist eine einfache Ausbildung des Indikatorbereichs möglich.

Vorzugsweise sind die Messmarkierungen in Umfangsrichtung entlang des Führungselements angebracht, sodass ein Parallaxefehler beim Ablesen des Drehmoments vermieden und das aufgebrachte Drehmoment genau abgelesen werden kann.

In einer bevorzugten Ausführungsform begrenzt das Führungselement die Aussparung auf der dem Kopfbereich abgewandten Seite. Konkret ist das Führungselement durch eine erste Führungswand und eine zweite Führungswand gebildet, welche gegenüberliegend beabstandet voneinander und parallel zur Ebene verlaufen. Der Abstand zwischen diesen ist derart bemessen, dass der Betätigungshebel frei ausgelenkt werden kann. Ferner stehen der erste Führungswand und der zweite Führungswand vom freien Endbereich des Schaftbereichs ab. Somit ist der Betätigungshebel bei seiner Auslenkung in der Auslenkungsebene sicher gehalten und kann nicht seitlich gebogen werden. Weiter kann der Betätigungshebel im Mundbereich nicht einhaken, sodass die Bedienung des Drehmomentschlüssels sicher ist. Diese Ausführungsform ist insbesondere auch vorteilhalt, weil sich der Betätigungshebel nicht in andere Werkzeuge bei der Reinigung oder bei der Benutzung verhakt. Somit kann eine Verformung des Betätigungshebels vermieden werden und eine zuverlässige Benutzung gewährleistet werden.

In einer bevorzugten Ausführungsform sind die Messmarkierungen des Indikatorbereichs auf der ersten Führungswand und/oder auf der zweiten Führungswand angebracht. Somit wird sichergestellt, dass der Betätigungshebel in der Auslenkungsebene eine relative Position bezüglich der Messmarkierungen behält, welche das Risiko eines Parallaxefehlers beim Ablesen des Drehmoments reduziert.

Zur Herstellung des Drehmomentschlüssels sind Werkstoffe geeignet, welche bereits in der Medizinaltechnik eingesetzt werden und biokompatibel, reinigbar sowie sterilisierbar sind. Geeignete Materialien sind hierbei beispielsweise Metall und Metalllegierungen wie Stahl, rostfreier Stahl, Titan, Titanlegierungen, und Kunststoff.

Der Drehmomentschlüssel kann durch Spritzgiessen oder Fräsen hergestellt werden.

Der Drehmomentschlüssel kann auch zum Lösen des Implantats bzw. des Verbindungselements gebraucht werden, indem der Drehmomentschlüssel um den Betätigungshebel um 180° gedreht wird und auf das Eindrehinstrument wieder aufgesetzt wird.

Weitere Vorteile und Eigenschaften der Erfindung gehen aus der nachstehenden Beschreibung eines Ausführungsbeispiels hervor, welches anhand der beiliegenden Figuren erläutert wird.

Diese zeigen rein schematisch:
- Fig. 1: eine Draufsicht des erfindungsgemässen Drehmomentschlüssels, dessen Betätigungshebel in seiner Ruheposition liegt;
- Fig. 2: eine Rückansicht des Drehmomentschlüssels gemäss Fig. 1;
- Fig. 3: eine Rückansicht des Drehmomentschlüssels gemäss Fig. 1 bei der Ausübung einer Referenzkraft auf den Betätigungshebel;
- Fig. 4: eine Rückansicht des Drehmomentschlüssels gemäss Fig. 1 bei der Ausübung einer die Referenzkraft überschreitenden Kraft auf den Betätigungshebel;
- Fig. 5: eine Darstellung des ausgeübten Drehmoments in Abhängigkeit von der Auslenkung des Betätigungshebels für den Drehmomentschlüssel gemäss Fig. 1, sowie für die in EP 0 704 281 A1 und DE 20 2004 014 195 U1 offenbarten Drehmomentschlüssel;
- Fig. 6: eine Draufsicht einer weiteren Ausführungsform des erfindungsgemässen Drehmomentschlüssels, dessen Betätigungshebel in der Ruheposition liegt;
- Fig. 7: eine Seitenansicht des Drehmomentschlüssels gemäss Fig. 6 auf dessen dem Indikatorbereich zugewandten Seite; und
- Fig. 8: eine Seitenansicht in Längsrichtung des Drehmomentschlüssels gemäss Fig. 6.

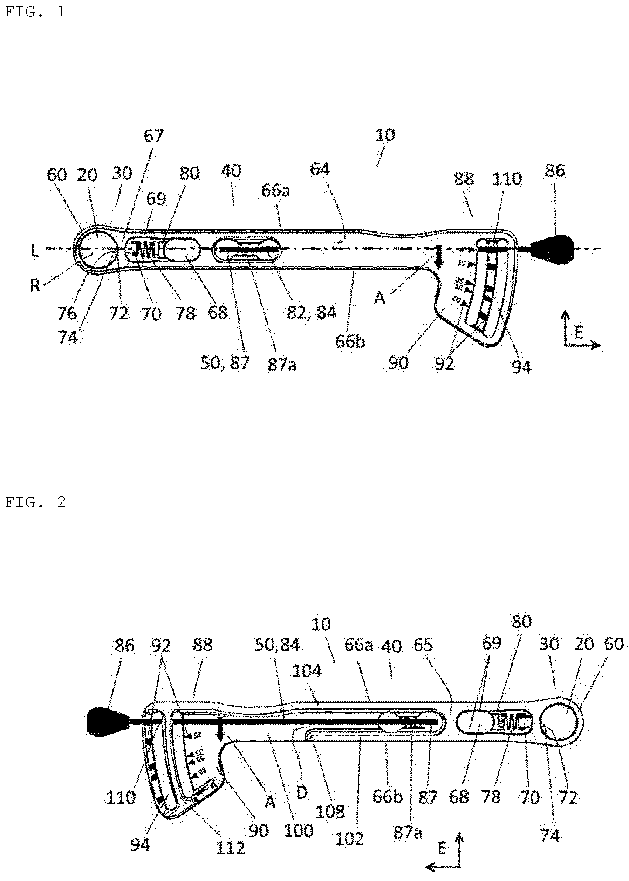

Der in der Fig. 1 bis 4 abgebildete Drehmomentschlüssel 10 umfasst einen eine Aufnahmeöffnung 20 aufweisenden Kopfbereich 30, einen an den Kopfbereich 30 anschliessenden, eine Längsachse L des Drehmomentschlüssels definierenden, wenigstens annähernd biegefesten Schaftbereich 40, und einen am Schaftbereich 40 befestigten, stabförmigen, im Querschnitt kreisförmigen, elastisch biegbaren Betätigungshebel 50 zum Aufbringen eines Drehmoments auf den Kopfbereich 30. Der Schaftbereich ist derart ausgebildet, dass die Längsachse L die Rotationsachse schneidet.

Die Aufnahmeöffnung 20 des Kopfbereichs 30 ist durch eine kreisringförmige Umfassung 60 gebildet, welche eine Rotationsachse R definiert. Die Umfassung 60 ist dazu bestimmt, ein Eindrehinstrument in der Erstreckung der Rotationsachse R aufzunehmen. Eine Ebene E erstreckt sich rechtwinklig zur Rotationsachse R und umfasst eine Vorderseite 64 des Drehmomentschlüssels 10. In den Fig. 2 bis 4 ist eine Rückseite 65 des Drehmomentschlüssels 10 ersichtlich, welche sich wenigstens annähernd parallel zur Ebene E erstreckt. Der Drehmomentschlüssel 10 ist seitlich durch wenigstens annähernd rechtwinklig zur Ebene E verlaufende Seitenflächen 66a und 66b begrenzt.

Der Kopfbereich 30 weist eine Ratschenfunktion auf, welche einen Freilauf des eingesetzten Eindrehinstruments bei einer Drehung entgegen einer Anzugsrichtung A ermöglicht. Zur Verwirklichung der Ratschenfunktion in der gezeigten Ausführungsform weist der Kopfbereich 30 eine wenigstens annähernd zentriert zur Längsachse L verlaufende, kreiszylindrische Längsbohrung 67 auf, welche in die Aufnahmeöffnung 20 mündet. Das der Aufnahmeöffnung 20 abgewandte Ende der Längsbohrung 67 mündet in eine in den Schaftbereich 40 ausgebildete, sich entlang der Längsachse L erstreckende, längliche Aussparung 68, welche parallele Längsseiten 69 und halbkreisförmige Enden aufweist.

Ein kreiszylindrischer, in der Längsbohrung 67 passgenau gelagerter Stift 70 bildet auf seinem der Aufnahmeöffnung 20 zugewandten Endbereich eine Rastnase 72, welche eine wenigstens annähernd radial verlaufende Mitnahmefläche 74 aufweist. Die Mitnahmefläche 74 ist dazu bestimmt, mit einer wenigstens annähernd radial zur Rotationsachse R verlaufenden Gegenfläche des eingesetzten Eindrehinstruments zusammenzuwirken. Weiter weist die Rastnase eine, in Anzugsrichtung A gesehen, der Mitnahmefläche 74 nachlaufende, direkt anschliessende Angriffsfläche 76 auf, welche die Bewegung der Rastnase 74 in ihre Freigabestellung und folglich einen Freilauf des Eindrehinstruments erlaubt. Die Angriffsfläche 76 ist durch eine Abschrägung 76 gebildet, deren Abstand zur Rotationsachse R entgegen der Anzugsrichtung A zunimmt.

Auf seinem der Aufnahmeöffnung 20 abgewandten Endbereich ist der Stift 70 an einer sich zentriert zur Längsachse L in der Aussparung 68 erstreckenden Feder 78 befestigt. Die Feder 78 ist mit ihrem dem Stift 70 abgewandten Ende an einem schräg zur Längsachse L fest angeordneten, sich zwischen den Längsseiten 69 erstreckenden Steg 80 befestigt.

Die Länge des Stifts 70, die Spannung der Feder 78 und die Position des Stegs 80 in der Aussparung 68 sind derart bemessen, dass die Rastnase 72 in ihrer Mitnahmestellung in die Aufnahmeöffnung 20 hineinragt und in ihrer Freigabestellung beim Drehen entgegen der Anzugsrichtung A in die Längsbohrung 67 hineingeschoben werden kann, um den Freilauf zu erlauben.

Der Betätigungshebel 50 umfasst einen elastisch biegbaren Biegeabschnitt 84 und einen an den Biegeabschnitt 84 anschliessenden Griffabschnitt 86. Der Betätigungshebel 50 ist über einen dem Griffabschnitt 86 abgewandten Endbereich 87 des Betätigungshebels 50 zwischen einem Auflager 108 und dem Kopfbereich 30 am Schaftbereich 40 befestigt. Der Griffabschnitt 86 ist vorliegend durch eine birnenförmige Verdickung ausgebildet, um einen besseren Griff des Betätigungshebels 50 zu ermöglichen. In der vorliegenden Ausführungsform ist der Endbereich 87 des Betätigungshebels 50 in einen in Längsrichtung L verlaufenden, verzahnten Klemmspalt 87a des Schaftbereichs 40 ortsfest geklemmt.

Ausserdem umfasst der Drehmomentschlüssel 10 einen auf das dem Kopfbereich 30 abgewandten Ende des Schaftbereichs 40 abstehenden Indikatorbereich 88, wobei der Griffabschnitt 86 sich über den Indikatorbereich 88 hinaus erstreckt.

Der Indikatorbereich 88 erstreckt sich in der Form eines Plättchens 90, dessen Vorderfläche in der Ebene E verläuft. Auf dem Indikatorbereich 88 sind Messmarkierungen 92 im Randbereich einer nachstehend näher beschriebenen Ausnehmung 94 angebracht, an welchen die Auslenkung des Betätigungshebels 50 als erzeugtes Drehmoment ablesbar ist.

Auf seiner Rückseite 65 weist der Schaftbereich 40 eine parallel zur Ebene E verlaufende, sich bis in den Indikatorbereich 88 erstreckende Aussparung 100 auf, in welcher der Betätigungshebel 50 verläuft und welche einen Freiraum für die Auslenkung des Betätigungshebels 50 bildet, wie dies auf Fig. 2 bis 4 ersichtlich ist. Die Tiefe der Aussparung 100 ist derart bemessen, dass der Betätigungshebel 50 in seiner Ruheposition zentriert zur Längsachse L verlaufen kann.

Die Aussparung 100 ist durch eine sich in Längsrichtung L erstreckende, in Anzugsrichtung A gesehen, dem Betätigungshebel 50 vorlaufende Wand 102 und dem Betätigungshebel 50 nachlaufende weitere Wand 104 begrenzt. Die weitere Wand 104 erstreckt sich über den Schaftbereich 40 und den Indikatorbereich 88. Hingegen erstreckt sich die Wand 102 nur über einen dem Kopfbereich 30 zugewandten Abschnitt des Schaftbereichs 40. Die der Aussparung 100 abgewandte Fläche der Wand 102 und der weiteren Wand 104 gehören zu den Seitenflächen 66b bzw. 66a.

Der Querschnitt des Schaftbereichs 40 in seinem zwischen einem Befestigungspunkt des Betätigungshebels 50 und dem Auflager 108 verlaufenden Abschnitt ist U-förmig ausgebildet, wobei der Boden der U-Form den Boden der Aussparung 100 bildet. Vorliegend ist der Befestigungspunkt durch das der Aussparung zugewandte Ende des Klemmspalts 87a gebildet. Von seiner Ruheposition bis zu einer ausgelenkten Position bewegt sich der Betätigungshebel 50, in Querschnitt gesehen, in den Freiraum zwischen den zwei Schenkeln der U-Form.

Weiter ist der Querschnitt des Schaftbereichs 40 in seinem zwischen dem Auflager 108 und dem dem Indikatorbereich 88 zugewandten Ende des Schaftbereichs 40 verlaufenden Abschnitt L-förmig ausgebildet, wobei ein Schenkel der L-Form den Boden der Aussparung 100 bildet und der weitere Schenkel der L-Form dem Betätigungshebel 50, in Anzugsrichtung gesehen, nachlaufend ausgebildet ist.

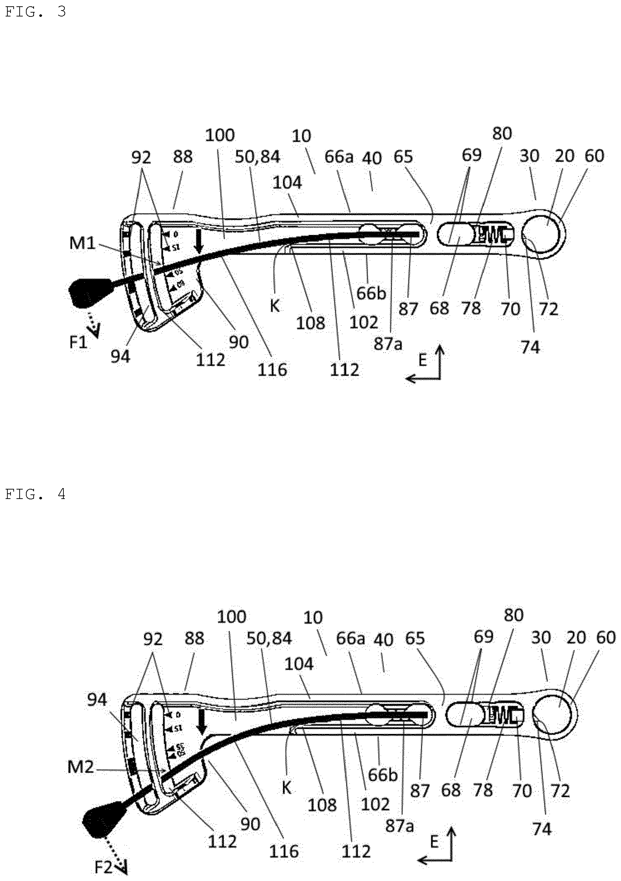

Der dem freien Ende des Betätigungshebels 50 zugewandter Endbereich 108 der Wand 102 bildet das Auflager 108. Der Abstand D, in Umfangsrichtung bezüglich der Rotationsachse R gesehen, zwischen dem Betätigungshebel 102 und dem Auflager 108 ist derart bemessen, dass beim Erreichen einer Referenzkraft F1 der ausgelenkte Betätigungshebel 50 am Auflager 108 in Anlage kommt, wie dies auf Fig. 3 gezeigt ist. Der Referenzkraft F1 entspricht ein Referenzdrehmoment M1, welches auf das Eindrehinstrument ausgeübt wird.

Im Indikatorbereich 88 steht die Aussparung 100 in Verbindung mit der Ausnehmung 94 über die gesamte Erstreckung der Ausnehmung 94. Die Ausnehmung 94 weist die Form eines konvex zur Rotationsachse R verlaufenden Kreisringsegments auf, welches Kreisringsegment sich in Umfangsrichtung derart erstreckt, dass ein Indikatorabschnitt 110 des Betätigungshebels 50 durch die Ausnehmung 94 von seiner Ruheposition bis zu seiner maximal ausgelenkten Position sichtbar ist. Somit ist das erzeugte Drehmoment über die Auslenkung des Betätigungshebels 50, insbesondere über die Bewegung des Indikatorabschnitts 110 gegenüber die am Randbereich der Ausnehmung 94 in Umfangsrichtung angebrachten Messmarkierungen 92 ablesbar.

Die Rückseite 66 des Drehmomentschlüssels 10 weist einen, in Umfangsrichtung gesehen, über die gesamte Erstreckung der Ausnehmung 94 parallel zur Ebene E verlaufenden Steg 112 auf, welcher die Seitenflächen 66a bzw. 66b verbindet. Der Betätigungshebel 50 ist zwischen dem Steg 112 und einem Boden der Aussparung 100 angeordnet, sodass der Steg 112 zusammen mit dem Boden der Aussparung 100 ein Führungselement für den Betätigungshebel 50 bildet. Somit verläuft die Auslenkung des Betätigungshebels 50 in einer Auslenkungsebene parallel zur Ebene E.

Vorliegend sind die Umfassung 60, der Schaftbereich 40, das Auflager 108 und der Indikatorbereich 88 einstückig ausgebildet.

Wenn eine in Anzugsrichtung A gerichtete, die Referenzkraft F1 überschreitende Kraft F2 ausgeübt wird, wie in Fig. 4 gezeigt, ändert sich das Biegeverhalten des Biegeabschnitts 84.

Ein erster Bogenabschnitt 114, welcher vom Endbereich 87 des Biegeabschnitts 84 bis zu einem Kontaktpunkt K des Biegeabschnitts 84 mit dem Auflager 108 verläuft, behält annähernd die gleiche Biegung wie beim Erreichen des Auflagers 108. In der gezeigten Ausführungsform entspricht die Länge des ersten Bogenabschnitts 114 etwa ein Drittel der Länge des Biegeabschnitts 84.

Da die Biegung des ersten Bogenabschnitts 114 in der gezeigten Ausführungsform relativ klein bleibt, kann die Position des Auflagers 108 ebenfalls um etwa ein Drittel der Länge des Biegeabschnitts 84, in Längsrichtung gesehen und gemessen ab dem dem Kopfbereich 30 zugewandten Ende des Biegeabschnitts 84, d.h. gemessen ab dem Befestigungspunkt, geschätzt werden.

Hingegen verformt und biegt sich ein zweiter Bogenabschnitt 116, welcher von dem Kontaktpunkt K bis zu dem dem Kopfbereich 30 abgewandten Ende des Biegeabschnitts 84 verläuft, weiter in Anzugsrichtung A in Abhängigkeit von der ausgeübten Kraft F2. Das dieser höheren Kraft F2 entsprechende Drehmoment M2 kann somit durch Ablesen auf dem Indikatorbereich 88 weiter in einer kontrollierbaren Weise ausgeübt werden. Ferner erfolgt die Auslenkung des Betätigungshebels 50 hauptsächlich durch die Auslenkung des zweiten Bogenabschnitts 116, sodass kein zusätzlicher Platz für die Auslenkung und weitere Verformung des ersten Bogenabschnitts 114, welche innerhalb des Freiraums im Schaftbereich erfolgen, benötigt wird.

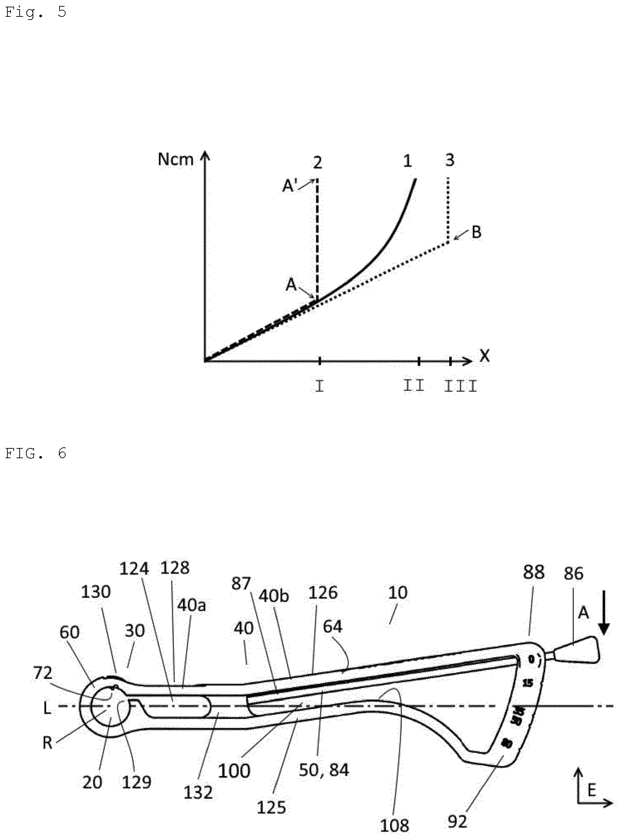

Auf der Grafik der Fig. 5 ist das ausgeübte Drehmoment auf der Y-Achse (in Ncm) in Abhängigkeit von der Auslenkung des Betätigungshebels auf der X-Achse für den Drehmomentschlüssel gemäss Fig. 1 (Kurve 1), sowie für die in EP 0 704 281 A1 oder DE 20 2004 014 195 U1 offenbarten Drehmomentschlüssel schematisch dargestellt.

Mit dem in DE 20 2004 014 195 U1 oder EP 0 704 281 A1 offenbarten Drehmomentschlüssel kann das ausgeübte Drehmoment durch die Auslenkung des Betätigungshebels abgelesen werden, bis der Betätigungshebel das Auflager erreicht hat. Dies entspricht dem Bereich der Kurve 2, welcher zwischen dem Ursprung der X- und Y-Achse und dem Punkt A verläuft, wobei der Punkt A einer Auslenkung I entspricht. Nachdem der Betätigungshebel die Auslenkung I erreicht hat und somit am Auflager in Anlage gekommen ist, kann ein höheres Drehmoment durch Ausübung einer höheren Kraft auf den Betätigungshebel ausgeübt werden, wie dies durch den sich vom Punkt A bis A' erstreckenden Abschnitt der Kurve 2 veranschaulicht ist. Allerdings kann dieses Drehmoment nicht mehr durch die Auslenkung des Betätigungshebels abgelesen werden, welche nämlich auf den durch den Punkt I materialisierten Wert blockiert bleibt.

Wie vorstehend bei der Würdigung dieser Dokumente erwähnt, kann ein höheres und ablesbares Drehmoment erreicht werden, wie in der Fig. 5 nun durch die Kurve 3 veranschaulicht, wenn der Betätigungshebel erst bei grösseren Auslenkungen, wie in der Fig. 5 nun durch den Punkt III materialisiert, in Anlage am Auflager kommt. Dies veranschaulicht die Kurve 3 für einen entsprechend geänderten Drehmomentschlüssel, welcher ein ablesbares Drehmoment bis zum durch den Punkt B materialisierten Drehmomentwert erlaubt. In diesem Fall muss jedoch mehr Freiraum für die Auslenkung des Betätigungshebels vorhanden sein.

Mit dem erfindungsgemässen Drehmomentschlüssel kann das ausgeübte Drehmoment auch abgelesen werden, nachdem der Betätigungshebel das Auflager erreicht hat. Dies veranschaulicht die Kurve 1, wobei die Auslenkung des Betätigungshebels bis zum Erreichen des Auflagers den Bereich der Kurve 1 entspricht, welcher zwischen dem Ursprung der X- und Y-Achse und dem Punkt A verläuft.

Wenn der Betätigungshebel weiter ausgelenkt wird, ändert sich das Biegeverhalten des Biegeabschnitts, wie dies vorstehend erklärt wurde. Der erste Bogenabschnitt behält annähernd die gleiche Biegung wie beim Erreichen des Auflagers, sodass kein zusätzlicher Freiraum für die Auslenkung des ersten Bogenabschnitts bei der weiteren Auslenkung des Betätigungshebels benötigt wird. Der zweite Bogenabschnitt kann weiter in Anzugsrichtung A in Abhängigkeit der ausgeübten Kraft gebogen werden. Dabei bleibt das ausgeübte Drehmoment ablesbar, wie dies vorstehend ebenfalls erklärt wurde. Für das gleiche ausgeübte, ablesbare Drehmoment ist jedoch der für die Auslenkung des Betätigungshebels, insbesondere des ersten Bogenabschnitts, benötigte Freiraum kleiner (Punkt II) für den erfindungsgemässen Drehmomentschlüssel als für die in EP 0 704 281 A1 oder DE 20 2004 014 195 U1 offenbarten Drehmomentschlüssel (Punkt III).

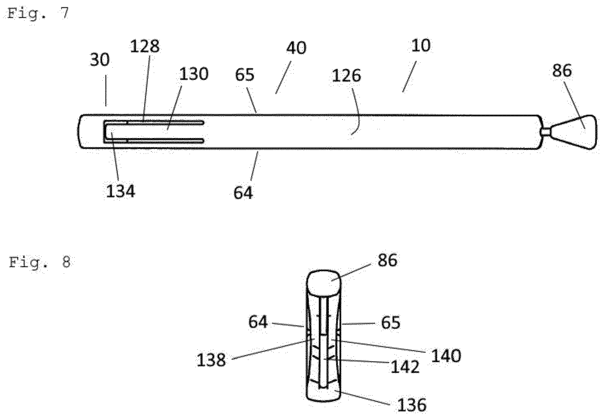

In den Fig. 6, 7 und 8 ist eine weitere Ausführungsform des Drehmomentschlüssels dargestellt, wobei für gleich wirkende Teile im Folgenden dieselben Referenznummern wie bei der ersten Ausführungsform verwendet werden. Des Weiteren ist diese weitere Ausführungsform ähnlich wie die erste Ausführungsform aufgebaut. Daher werden nachfolgend hauptsächlich die Unterschiede beschrieben.

Wie in Fig. 6 ersichtlich umfasst der Schaftbereich 40 einen sich in Längsrichtung L erstreckenden, an den Kopfbereich 30 anschliessenden, stabförmigen ersten Abschnitt 40a, dessen Querschnitt im Wesentlichen rechteckig ist und konstant über die Länge des ersten Abschnitts 40a bleibt. Ferner umfasst der Schaftbereich 40 einen an den ersten Abschnitt 40a anschliessenden, sich in der Ebene E aufweitend ausgebildeten zweiten Abschnitt 40b. Der zweite Abschnitt 40b weist eine von der Vorderseite 64 des Drehmomentschlüssels 10 zur Rückseite 65 durchgehende Aussparung 100 auf, in welcher der Betätigungshebel 50 verläuft und welche einen Freiraum für die Auslenkung des Betätigungshebels 50 bildet. Auf dem freien Ende des zweiten Abschnitts 40b ist der Indikatorbereich 88 ausgebildet, welcher im Wesentlichen in einer umfänglichen Richtung bezüglich der Rotationsachse R verläuft.

Die Aussparung 100 ist durch eine, in Anzugsrichtung A gesehen, dem Betätigungshebel 50 nachlaufende Seitenwand 126 des Schaftbereichs 40 und dem Betätigungshebel 50 vorlaufende weitere Seitenwand 125 des Schaftbereichs 40 sowie den die Seitenwand 126 und die weitere Seitenwand 125 am freien Ende des zweiten Abschnitts 40b verbindenden Indikatorbereich 88 ausgebildet.

Der Kopfbereich 30 weist ebenfalls eine Ratschenfunktion auf, welche einen Freilauf des eingesetzten Eindrehinstruments bei einer Drehung entgegen einer Anzugsrichtung A ermöglicht. Zur Verwirklichung der Ratschenfunktion umfasst der erste Abschnitt 40a eine sich in Längsrichtung L erstreckende, von der Vorderseite 64 des Drehmomentschlüssels 10 zur Rückseite 65 durchgehende, längliche Öffnung 124, welche durch die Seitenwand 126 und die im ersten Abschnitt 40a zur Seitenwand 126 parallele weitere Seitenwand 125 begrenzt ist. Die Öffnung 124 mündet über einen von der Vorderseite 64 zur Rückseite 65 durchgehenden Schlitz 129 in die Aufnahmeöffnung 20. Ferner erstreckt sich eine in Richtung zur Ebene E parallele, durchgehende weitere Öffnung 128 durch die Seitenwand 126 und den Kopfbereich 30, welche in Verbindung mit der Aufnahmeöffnung 20 und dem Schlitz 129 sowie die Öffnung 124 steht. Die weitere Öffnung 128 ist im Wesentlichen U-förmig, wobei der Boden der U-Form im Kopfbereich 30 liegt und die zwei Schenkeln der U-Form im ersten Abschnitt 40a teilweise verlaufen.

Die weitere Öffnung 128 begrenzt eine integral mit dem Schaftbereich 40 zwischen den zwei Schenkeln der U-Form ausgebildete Federzunge 130. Auf dem freien Ende 134 der Federzunge 130, d.h. auf der dem Boden der U-Form zugewandten Seite der Federzunge 130, umfasst die Federzunge 130 die Rastnase 72. Die Federzunge 130 ist derart ausgebildet, dass die Rastnase 72 in ihrer Mitnahmestellung in die Aufnahmeöffnung 20 hineinragt und in ihrer Freigabestellung beim Drehen entgegen der Anzugsrichtung A in die weitere Öffnung 128 hineingeschoben werden kann, um den Freilauf zu erlauben.

Die Federkraft der Federzunge 130 kann über ihre Dicke, Länge und Breite eingestellt werden.

Die Öffnung 124 ist auf ihrer dem Kopfbereich abgewandten Seite durch einen Steg 132 begrenzt und halbkreisförmig ausgebildet, wobei der Steg 132 einen Endbereich des ersten Abschnitts 40a bildet und die Seitenwand 126 und die weitere Seitenwand 125 verbindet.

Wie in der ersten Ausführungsform, umfasst der Betätigungshebel 50 den elastisch biegbaren Biegeabschnitt 84 und den an den Biegeabschnitt 84 anschliessenden, birnenförmigen Griffabschnitt 86, welcher sich über den Indikatorbereich 88 hinaus erstreckt. Der Betätigungshebel 50 ist über einen dem Griffabschnitt 86 abgewandten Endbereich 87 des Betätigungshebels 50 zwischen dem Auflager 108 und dem Kopfbereich 30 am Schaftbereich 40 befestigt. Vorliegend ist der Betätigungshebel 50 in eine in den Steg 132 im Wesentlichen radial zur Rotationsachse R ausgebildete Bohrung ortsfest geklemmt. Somit ist der Betätigungshebel 50 in Längsrichtung L weg vom Kopfbereich 30 zur Rotationsachse R radial versetzt befestigt.

In seiner Ruheposition ist der Betätigungshebel 50 in der Ebene E schräg zur Längsrichtung L angeordnet, in einem spitzen Winkel von ca. 15 Grad entgegen der Anzugsrichtung A von der Längsrichtung L zum Betätigungshebel 50 gemessen. Vorliegend erstreckt sich die Seitenwand 126 im zweiten Abschnitt 40b parallel zur Ruheposition des Betätigungshebels 50.

Die weitere Seitenwand 125 weist im zweiten Abschnitt 40b eine konkav zur Rotationsachse R ausgebildete, in die Ebene E verlaufende Krümmung auf, welche das Auflager 108 bildet.

Konkret ist mindestens ein Abschnitt der weiteren Seitenwand 125 derart in Richtung zum Betätigungshebel 50 gebogen, dass, bei der Ausübung der in Anzugsrichtung A gerichteten Referenzkraft F1 auf den Betätigungshebel 50, der Biegeabschnitt 84 des Betätigungshebels 50 sich bogenförmig verformt und in Anlage an dem das Auflager bildenden Kontaktpunkt K kommt.

Bei der Aufbringung einer in Anzugsrichtung gerichteten, die Referenzkraft F1 überschreitenden Kraft kann der Biegeabschnitt 84 weiter in Anzugsrichtung gebogen werden, weil das Auflager ein fester Stützpunkt zum Biegen des Betätigungshebels bildet, wenn der Betätigungshebel 50 in Anlage mit dem Auflager kommt.

Der Indikatorbereich 88 begrenzt die Aussparung 100 auf der dem Kopfbereich 30 abgewandten Seite des Schaftbereichs 40 und bildet ein Führungselement 136 für den Betätigungshebel 50. Konkret ist das Führungselement 136 durch eine erste Führungswand 138 und eine zweite Führungswand 140 gebildet, welche gegenüberliegend beabstandet voneinander und parallel zur Ebene E verlaufen. Die erste Führungswand 138 und die zweite Führungswand 140 stehen vom freien Endbereich des Schaftbereichs 40 ab. Somit ist der Betätigungshebel 50 bei seiner Auslenkung in einem parallel zur Auslenkungsebene verlaufenden Führungsschlitz 142 sicher gehalten.

**Bezugszeichen**

| | |
|---|---|
| Ebene | E |
| Rotationsachse | R |
| Anzugsrichtung | A |
| Drehmomentschlüssel | 10 |
| Aufnahmeöffnung | 20 |
| Kopfbereich | 30 |
| Schaftbereich | 40 |
| Erster und zweiter Abschnitt 40a bzw. 40b des Schaftbereichs | 40 |
| Betätigungshebel | 50 |
| Umfassung | 60 |
| Eindrehinstrument | 62 |
| Vorderseite | 64 |
| Rückseite | 65 |
| Seitenfläche | 66a, 66b |
| Längsbohrung | 67 |
| Aussparung | 68 |
| Längsseiten (der Aussparung) | 69 |
| Stift | 70 |
| Rastnase | 72 |
| Mitnahmefläche | 74 |
| Angriffsfläche | 76 |
| Feder | 78 |
| Steg | 80 |
| Biegeabschnitt | 84 |
| Griffabschnitt | 86 |
| Endbereich des Biegeabschnitts | 87 |
| Klemmspalt | 87a |
| Indikatorbereich | 88 |
| Plättchen | 90 |
| Messmarkierungen | 92 |
| Ausnehmung | 94 |
| Aussparung | 100 |
| Wand | 102 |
| weitere Wand | 104 |
| Auflager | 108 |
| Indikatorabschnitt des Betätigungshebels | 110 |
| Steg | 112 |
| erster Bogenabschnitt des Betätigungshebels | 114 |
| zweiter Bogenabschnitt des Betätigungshebels | 116 |
| Öffnung | 124 |
| weitere Seitenwand | 125 |
| Seitenwand | 126 |
| weitere Öffnung | 128 |
| Schlitz | 129 |
| Federzunge | 130 |
| Steg | 132 |
| freies Ende 134 der Federzunge | 130 |
| Führungselement | 136 |
| erste und zweite Führungswand 138 bzw. 140 des Führungselement | 136 |
| Führungsschlitz | 142 |
| Referenzdrehmoment | M1 |
| Referenzkraft | F1 |
| Drehmoment | M2 |
| Kraft | F2 |
| Kontaktpunkt | K |

## Patentansprüche

1. Drehmomentschlüssel (10) für die Medizinaltechnik, insbesondere die Dentalmedizin, mit einem eine Aufnahmeöffnung (20) aufweisenden Kopfbereich (30), welche Aufnahmeöffnung (20) eine Rotationsachse (R) definiert und dazu bestimmt ist, ein Eindrehinstrument in der Erstreckung der Rotationsachse (R) aufzunehmen, einem an den Kopfbereich (30) anschliessenden, sich wenigstens annähernd in einer rechtwinklig zur Rotationsachse (R) verlaufenden Ebene (E) erstreckenden, eine Längsachse (L) des Drehmomentschlüssels (10) definierenden, wenigstens annähernd biegefesten Schaftbereich (40), einem am Schaftbereich (40) ausgebildeten Auflager (108), und einem einen elastisch biegbaren Biegeabschnitt (84) und einen an den Biegeabschnitt (84) anschliessenden Griffabschnitt (86) umfassenden Betätigungshebel (50), welcher zwischen dem Auflager (108) und dem Kopfbereich (30) am Schaftbereich (40) mit einem dem Griffabschnitt (86) abgewandten Endbereich (87) des Betätigungshebels (50) befestigt ist, wobei in seiner Ruheposition der Betätigungshebel (50), in Umfangsrichtung bezüglich der Rotationsachse (R) gesehen, vom Auflager (108) beabstandet ist, der Betätigungshebel (50) bei der Aufbringung einer Kraft in einer Anzugsrichtung (A) auf dessen Griffabschnitt (86) bei in die Aufnahmeöffnung (20) eingesetztem Eindrehinstrument durch Mitnahme ein Drehmoment ans Eindrehinstrument überträgt und beim Erreichen einer Referenzkraft (F1) am Auflager (108) in Anlage kommt, wobei der Schaftbereich (40) eine parallel zur Ebene (E) verlaufende Aussparung (100) aufweist, in welcher der Betätigungshebel (50) verläuft und welche einen Freiraum für die Auslenkung des Betätigungshebels (50) bildet, **dadurch gekennzeichnet, dass** der Schaftbereich (50) eine sich in Längsrichtung (L) erstreckende, in Anzugsrichtung (A) gesehen, dem Betätigungshebel (50) vorlaufende Wand (102) aufweist, welche die Aussparung (100) begrenzt und eine das Auflager (108) bildende, konkav zur Rotationsachse (R) ausgebildete, in die Ebene (E) verlaufende Krümmung aufweist, und, dass beim Erreichen der Referenzkraft (F1) der Biegeabschnitt (84) am Auflager (108) in Anlage kommt, und der Biegeabschnitt (84) bei der Aufbringung einer in Anzugsrichtung (A) gerichteten, die Referenzkraft (F1) überschreitenden Kraft weiter in Anzugsrichtung (A) biegbar ist.

2. Drehmomentschlüssel (10) nach Anspruch 1, **dadurch gekennzeichnet, dass** das Auflager (108) fest am Schaftbereich (40) ausgebildet ist.

3. Drehmomentschlüssel (10) nach Anspruch 1 oder 2, **dadurch gekennzeichnet, dass** der Biegeabschnitt (84) durch einen Biegestab gebildet ist.

4. Drehmomentschlüssel (10) nach einem der Ansprüche 1 bis 3, **dadurch gekennzeichnet, dass** der Betätigungshebel (50) in seiner Ruheposition wenigstens annähernd radial zur Rotationsachse (R) und vorzugsweise parallel zur Längsrichtung (L) angeordnet ist.

5. Drehmomentschlüssel (10) nach einem der Ansprüche 1 bis 3, **dadurch gekennzeichnet, dass** in seiner Ruheposition der Betätigungshebel (50), in Längsrichtung (L) gesehen, weg vom Kopfbereich (30) radial versetzt zur Rotationsachse (R) und schräg zur Längsrichtung (L) angeordnet ist.

6. Drehmomentschlüssel (10) nach einem der Ansprüche 1 bis 5, **dadurch gekennzeichnet, dass** der Kopfbereich (30) eine Ratschenfunktion aufweist und einen Freilauf des eingesetzten Eindrehinstruments bei einer Drehung entgegen der Anzugsrichtung (A) ermöglicht.

7. Drehmomentschlüssel (10) nach einem der Ansprüche 1 bis 6, **dadurch gekennzeichnet, dass** bei der Aufbringung der Kraft in Anzugsrichtung (A) die Auslenkung des Betätigungshebels (50), ausgehend von dessen Ruheposition, ein Mass für das generierte Drehmoment ist.

8. Drehmomentschlüssel (10) nach einem der Ansprüche 1 bis 7, **gekennzeichnet durch** einen vom freien Endbereich des Schaftbereichs (40) abstehenden Indikatorbereich (88), wobei der Betätigungshebel (50) sich mindestens bis zum Indikatorbereich (88), bevorzugt bis in den Indikatorbereich (88) hinein, besonders bevorzugt über diesen hinaus erstreckt, wobei Messmarkierungen auf dem Indikatorbereich (88) angebracht sind, an welchen die Auslenkung des Betätigungshebels (50) als erzeugtes Drehmoment ablesbar ist.

9. Drehmomentschlüssel (10) nach einem der Ansprüche 1 bis 8, **dadurch gekennzeichnet, dass** der Betätigungshebel (50) durch ein Führungselement (136) in einer parallel zur Ebene (E) verlaufenden Auslenkungsebene bewegbar gehalten wird.

10. Drehmomentschlüssel (10) nach Anspruch 9, **dadurch gekennzeichnet, dass** der Indikatorbereich (88) integral mit dem Führungselement (136) ausgebildet ist.

11. Drehmomentschlüssel (10) nach einem der Ansprüche 9 oder 10, **dadurch gekennzeichnet, dass** das Führungselement (136) durch eine erste Führungswand (138) und eine zweite Führungswand (140) gebildet ist, welche vom freien Endbereich des Schaftbereichs (40) abstehen, gegenüberliegend beabstandet voneinander und parallel zur Ebene (E) verlaufen, und zwischen welchen der Betätigungshebel (50) frei ausgelenkt werden kann.

12. Drehmomentschlüssel (10) nach einem der Ansprüche 1 bis 11, **dadurch gekennzeichnet, dass** das Auflager (108), in Längsrichtung (L) gesehen, zwischen ein Viertel und zwei Drittel der Länge des Biegeabschnitts, bevorzugt etwa mittig zum Biegeabschnitt (84), besonders bevorzugt etwa um ein Drittel der Länge des Biegeabschnitts (84), gemessen ab dem dem Kopfbereich zugewandten Ende des Biegeabschnitts (84), angeordnet ist.

## Claims

1. A torque wrench (10) for the medical field, in particular for dentistry, with a head region (30) which has a receiving opening (20), which receiving opening (20) defines a rotation axis (R) and is intended to receive a screwing instrument in the extension of the rotation axis (R), an at least approximately flexurally resistant shaft region (40) which adjoins the head region (30), extends at least approximately in a plane (E) running at right angles to the rotation axis (R) and defines a longitudinal axis (L) of the torque wrench (10), with a support (108) formed on the shaft region (40), and with an actuating lever (50) which comprises an elastically bendable flexion portion (84) and a grip portion (86) adjoining the flexion portion (84), which actuating lever (50) is secured on the shaft region (40), between the support (108) and the head region (30), with an end region (87) of the actuating lever (50) facing away from the grip portion (86), wherein, in its rest position, the actuating lever (50), seen in a circumferential direction with respect to the rotation axis (R), is spaced from the support (108), and the actuating lever (50), when a force is applied to its grip portion (86) in a tightening direction (A), with the screwing instrument inserted in the receiving opening (20), transmits a torque by entrainment to the screwing instrument and, when a reference force (Fl) is reached, comes into contact with the support (108), wherein the shaft region (40) has a clearance (100) which runs parallel to the plane (E) and in which the actuating lever (50) runs, and which forms a free space for the deflection of the actuating lever (50), **characterized in that** the shaft region (50) has a wall (102) which extends in the longitudinal direction (L), which lies ahead of the actuating lever (50), as seen in the tightening direction (A), which delimits the clearance (100), and which has a curvature that forms the support (108) and that is concave to the rotation axis (R) and runs into the plane (E), and **in that**, when the reference force (Fl) is reached, the flexion portion (84) comes into contact with the support (108), and the flexion portion (84) is able to bend further in the tightening direction (A) upon application of a force that is directed in the tightening direction (A) and that exceeds the reference force (F1).

2. The torque wrench (10) as claimed in claim 1, **characterized in that** the support (108) is designed fixedly on the shaft region (40).

3. The torque wrench (10) as claimed in claim 1 or 2, **characterized in that** the flexion portion (84) is formed by a flexion rod.

4. The torque wrench (10) as claimed in one of claims 1 to 3, **characterized in that** the actuating lever (50), in its rest position, is arranged at least approximately radially with respect to the rotation axis (R) and preferably parallel to the longitudinal direction (L).

5. The torque wrench (10) as claimed in one of claims 1 to 3, **characterized in that**, in its rest position, the actuating lever (50), seen in the longitudinal direction (L), is arranged, away from the head region (30), radially offset with respect to the rotation axis (R) and oblique with respect to the longitudinal direction (L).

6. The torque wrench (10) as claimed in one of claims 1 to 5, **characterized in that** the head region (30) has a ratchet function and permits free running of the inserted screwing instrument upon rotation counter to the tightening direction (A).

7. The torque wrench (10) as claimed in one of claims 1 to 6, **characterized in that**, when the force is applied in the tightening direction (A), the deflection of the actuating lever (50), starting from its rest position, is a measure of the generated torque.

8. The torque wrench (10) as claimed in one of claims 1 to 7, **characterized by** an indicator region (88) protruding from the free end region of the shaft region (40), wherein the actuating lever (50) extends at least as far as the indicator region (88), preferably into the indicator region (88), particularly preferably beyond the latter, wherein measurement markings are applied to the indicator region (88), from which the deflection of the actuating lever (50) can be read off as generated torque.

9. The torque wrench (10) as claimed in one of claims 1 to 8, **characterized in that** the actuating lever (50) is held movably by a guide element (136) in a deflection plane running parallel to the plane (E).

10. The torque wrench (10) as claimed in claim 9, **characterized in that** the indicator region (88) is formed integrally with the guide element (136).

11. The torque wrench (10) as claimed in claims 9 or 10, **characterized in that** the guide element (136) is formed by a first guide wall (138) and a second guide wall (140) which protrude from the free end region of the shaft region (40) and run opposite to and spaced apart from each another and parallel to the plane (E), and between which the actuating lever (50) can be freely deflected.

12. The torque wrench (10) as claimed in one of claims 1 to 11, **characterized in that** the support (108), seen in the longitudinal direction (L), is arranged at between a quarter and two thirds of the length of the flexion portion, preferably approximately centrally with respect to the flexion portion (84), particularly preferably about a third of the length of the flexion portion (84), measured from that end of the flexion portion (84) facing toward the head region.

## Revendications

1. Clé dynamométrique (10) pour le domaine médical, en particulier pour la dentisterie, avec une zone de tête (30) qui présente une ouverture de réception (20), laquelle ouverture de réception (20) définit un axe de rotation (R) et est destinée à recevoir un instrument de vissage dans le prolongement de l'axe de rotation (R), une zone de manche (40) au moins approximativement résistante à la flexion, qui est adjacente à la zone de tête (30), s'étend au moins approximativement dans un plan (E) perpendiculaire à l'axe de rotation (R) et définit un axe longitudinal (L) de la clé dynamométrique (10), avec un support (108) formé sur la zone de manche (40), et avec un levier d'actionnement (50) qui comprend une partie de flexion (84) élastiquement pliable et une partie de préhension (86) adjacente à la partie de flexion (84), ce levier d'actionnement (50) étant fixé sur la zone de manche (40), entre le support (108) et la zone de tête (30), avec une région d'extrémité (87) du levier d'actionnement (50) orientée à l'opposé de la partie de préhension (86), dans laquelle, dans sa position de repos, le levier d'actionnement (50), vu dans une direction circonférentielle par rapport à l'axe de rotation (R), est espacé du support (108) et du levier d'actionnement (50), lorsqu'une force est appliquée à sa partie de préhension (86) dans une direction de serrage (A), avec l'instrument de vissage inséré dans l'ouverture de réception (20), transmet un couple par entraînement à l'instrument de vissage et, lorsqu'une force de référence (Fl) est atteinte, entre en contact avec le support (108), dans laquelle la zone de manche (40) présente un espace libre (100) parallèle au plan (E) dans lequel circule le levier d'actionnement (50) et qui forme un espace libre pour la flexion du levier d'actionnement (50), **caractérisée en ce que** la zone de manche (50) présente une paroi (102) qui s'étend dans la direction longitudinale (L), qui se trouve devant le levier d'actionnement (50), vu dans le sens de serrage (A), qui délimite l'espace libre (100), et qui a une courbure qui forme le support (108) et qui est concave par rapport à l'axe de rotation (R) et s'étend dans le plan (E), et **en ce que**, lorsque la force de référence (Fl) est atteinte, la partie de flexion (84) vient s'appuyer sur l'axe de rotation (R), la partie de flexion (84) entre en contact avec le support (108), et la partie de flexion (84) est capable de se plier davantage dans la direction de serrage (A) lors de l'application d'une force qui est dirigée dans la direction de serrage (A) et qui dépasse la force de référence (F1).

2. Clé dynamométrique (10) selon la revendication 1, **caractérisée par le fait que** le support (108) est conçu de manière fixe sur la zone de manche (40).

3. Clé dynamométrique (10) selon la revendication 1 ou 2, **caractérisée par le fait que** la partie de flexion (84) est formée par une tige de flexion.

4. Clé dynamométrique (10) selon l'une des revendications 1 à 3, **caractérisée en ce que** le levier d'actionnement (50), dans sa position de repos, est disposé au moins approximativement radialement par rapport à l'axe de rotation (R) et de préférence parallèlement à la direction longitudinale (L).

5. Clé dynamométrique (10) selon l'une des revendications 1 à 3, **caractérisée en ce que**, dans sa position de repos, le levier d'actionnement (50), vu dans la direction longitudinale (L), est disposé, à l'écart de la zone de tête (30), radialement décalé par rapport à l'axe de rotation (R) et oblique par rapport à la direction longitudinale (L) .

6. Clé dynamométrique (10) selon l'une des revendications 1 à 5, **caractérisée par le fait que** la zone de tête (30) a une fonction de cliquet et permet la libre circulation de l'instrument de vissage inséré lors d'une rotation dans le sens inverse du sens de serrage (A).

7. Clé dynamométrique (10) selon l'une des revendications 1 à 6, **caractérisée en ce que**, lorsque la force est appliquée dans le sens de serrage (A), la déviation du levier d'actionnement (50), à partir de sa position de repos, est une mesure du couple généré.

8. Clé dynamométrique (10) selon l'une des revendications 1 à 7, **caractérisée par** une zone d'indication (88) faisant saillie à partir de l'extrémité libre de la zone de manche (40), dans laquelle le levier d'actionnement (50) s'étend au moins jusqu'à la zone d'indication (88), de préférence dans la zone d'indication (88), de préférence encore au-delà de cette dernière, dans laquelle des marques de mesure sont appliquées sur la zone d'indication (88), à partir desquelles la déviation du levier d'actionnement (50) peut être lue en tant que couple généré.

9. Clé dynamométrique (10) selon l'une des revendications 1 à 8, **caractérisée par le fait que** le levier d'actionnement (50) est maintenu mobile par un élément de guidage (136) dans un plan de déviation parallèle au plan (E).

10. Clé dynamométrique (10) selon la revendication 9, **caractérisée en ce que** la zone indicatrice (88) est formée intégralement avec l'élément de guidage (136) .

11. Clé dynamométrique (10) selon la revendication 9 ou 10, **caractérisée en ce que** l'élément de guidage (136) est formé d'une première paroi de guidage (138) et d'une deuxième paroi de guidage (140) qui font saillie à partir de l'extrémité libre de la zone de manche (40) et qui sont opposées et espacées l'une de l'autre et parallèles au plan (E), et entre lesquelles le levier d'actionnement (50) peut être librement dévié.

12. Clé dynamométrique (10) selon l'une des revendications 1 à 11, **caractérisée en ce que** le support (108), vu dans la direction longitudinale (L), est disposé entre un quart et deux tiers de la longueur de la partie de flexion, de préférence approximativement au centre de la partie de flexion (84), de préférence encore à environ un tiers de la longueur de la partie de flexion (84), mesurée à partir de l'extrémité de la partie de flexion (84) orientée vers la région de tête.
